# EUROPEAN PATENT APPLICATION

(11) **EP 4 470 484 A1**
(43) Date of publication of application: **04.12.2024**
(21) Application number: 23747098.4
(22) Date of filing: 27.01.2023
(51) Int. Cl.: A61B 17/22, A61F 2/90

(54) **STENT RETRIEVER AND STENT**

(30) Priority: 28.01.2022 JP 2022012327
(71) Applicant: Nipro Corporation, Osaka 566-8510 (JP)
(72) Inventor: MATSUMOTO, Kuniaki, Osaka-shi, Osaka 531-8510 (JP); YASUMURA, Naoaki, Osaka-shi, Osaka 531-8510 (JP)
(74) Representative: Slingsby Partners LLP
(86) International application number: PCT/JP2023/002680
(87) International publication number: WO 2023/145888

(57) **Abstract**

To provide a stent retriever and a stent with a novel structure that can achieve excellent safety by preventing a marker from falling off while ensuring good visibility under the X-ray fluoroscopy with the marker.

A stent retriever 10 comprising: a self-expansion type linear skeleton 12 that becomes a mesh structure in an expanding state; a widened section 24 being partially widened in the linear skeleton 12; and a marker constituted by forming a plating layer 26 on both sides of the widened section 24. The selected drawing illustrating the stent retriever is just an example, and the present invention may be applied to various types of stents.

## Description

### TECHNICAL FIELD

The present invention relates to a stent retriever used for thrombus retrieval therapy to retrieve a thrombus in a blood vessel and a stent delivered into the blood vessel.

### BACKGROUND ART

Thrombus retrieval therapy using a catheter has conventionally been proposed as a method of removing an intravascular thrombus. Thrombus retrieval therapy is a treatment method to restore blood flow by delivering a stent retriever to a thrombus through the catheter inserted percutaneously into a blood vessel and retrieving the thrombus with the stent retriever.

The stent retriever has a self-expansion type linear skeleton that becomes a mesh structure in its expanding state, like a device for restoring blood flow disclosed in PCT Japanese Translation Patent Publication No. JP-A-2011-512900 (Patent Document 1), for example. The stent retriever expands at the site of thrombus formation in the blood vessel, and the mesh-structured linear skeleton entangles the thrombus, thereby retrieving it.

### BACKGROUND ART DOCUMENTS

### PATENT DOCUMENTS

Patent Document 1: JP-A-2011-512900

### SUMMARY OF THE INVENTION

### PROBLEM THE INVENTION ATTEMPTS TO SOLVE

By the way, since the stent retriever is delivered percutaneously through the catheter to the treatment site in the body, visibility under the X-ray fluoroscopy is required. However, the linear skeleton of the self-expansion type stent retriever made of Ni-Ti alloy or the like cannot ensure visibility under the X-ray fluoroscopy. Therefore, Patent Document 1 shows a structure equipped with radiopaque markers at its tip part (distal end) and base part (proximal end) of the stent retriever.

However, the inventor has examined the conventional structure described in Patent Document 1, and found that in order to attach the radiopaque markers to the distal and proximal ends of the stent retriever, it is necessary to attach markers that are separate from the linear skeleton of the stent retriever to the linear skeleton, and if the markers should fall off from the linear skeleton, it could cause serious consequences.

The present invention has been developed in view of the above-described matters as the background, and it is an object of the present invention to provide a stent retriever with a novel structure which is able to ensure good visibility under the X-ray fluoroscopy with a marker, while achieving excellent safety by preventing the marker from falling off.

A stent is also known as a therapeutic device that is delivered into a blood vessel in the same manner as the stent retriever for use, and similar issues exist with the stent. In other words, in the stent with a mesh structure, if a separate marker is attached to ensure visibility under the X-ray fluoroscopy, the marker has a risk of falling off.

### MEANS FOR SOLVING THE PROBLEM

The inventor first considered providing a radiopaque plating layer over the entire mesh structure of the stent retriever as a marker. However, the inventor found that the plating layer on the whole stent retriever is not practical because it significantly deteriorates the expansion/contraction and bending deformation characteristics and the like while increasing sliding resistance to the catheter, although visibility is good under the X-ray fluoroscopy. Therefore, the inventor, through repeated examination and experimentation, came up with the present invention, which can satisfy all of the required performance such as visibility, deformation characteristics, and sliding resistance.

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a stent retriever comprising: a self-expansion type linear skeleton that becomes a mesh structure in an expanding state; a widened section being partially widened in the linear skeleton; and a marker constituted by forming a plating layer on both sides of the widened section.

With the stent retriever structured according to the present preferred embodiment, the marker is constituted by the widened section provided in the linear skeleton, so that the marker does not fall off the linear skeleton and excellent safety is realized. In addition, since the marker is provided integrally with the linear skeleton, the number of parts can be reduced compared to the structure described in Patent Document 1, wherein the separate marker is fixed to the linear skeleton.

By forming the plating layer on the both sides of the widened section, the marker with excellent visibility under the X-ray fluoroscopy can be obtained. In particular, good visibility and more stable realization of the plating layer can be achieved by forming the plating layer on the widened section, which is made wider, to constitute the marker.

Since the widened section is partial in the linear skeleton, thus suppressing adverse effects on the mechanical properties of the stent retriever. This provides flexible deformation characteristics in the expansion, contraction, bending, etc. of the linear skeleton, while suppressing an increase in sliding resistance to the catheter or the like due to the plating layer.

A second preferred embodiment provides the stent retriever according to the first preferred embodiment, wherein the widened section constituting the marker includes a continuous widened part extending continuously over an entire length of the liner skeleton in a retriever length direction.

With the stent retriever structured according to the present preferred embodiment, the stent retriever in the blood vessel can be seen over its entire length by the marker constituted by the continuous widened part, under the X-ray fluoroscopy, which makes it easier to grasp the position and bending degree, etc. of the stent retriever.

A third preferred embodiment provides the stent retriever according to the first or second preferred embodiment, wherein the widened section constituting the marker includes a plurality of divided widened parts provided separately at multiple locations.

With the stent retriever structured according to the present preferred embodiment, the divided widened parts provided separately at the multiple locations can suppress the increase in deformation rigidity compared to the case where the widened part is provided continuously. This makes it possible to set the deformation characteristics of the linear skeleton more flexibly, for example.

A fourth preferred embodiment provides the stent retriever according to any one of the first through third preferred embodiments, wherein the widened section constituting the marker extends continuously in a retriever circumferential direction for half a circumference or more.

With the stent retriever structured according to the present preferred embodiment, it is easier to grasp the diameter of the linear skeleton when viewing the marker under the X-ray fluoroscopy. For example, it is possible to check whether the linear skeleton is properly expanding.

A fifth preferred embodiment provides the stent retriever according to any one of the first through fourth preferred embodiments, wherein at a proximal end of the linear skeleton, a single connecting line extends toward a control wire, and the widened section constituting the marker includes a proximal end widened part constituted by the connecting line.

With the stent retriever structured according to the present preferred embodiment, the proximal end position of the linear skeleton can be grasped under the X-ray fluoroscopy by viewing the connecting line. In addition, the connecting line can be provided at a location further to the proximal end side than the part of the linear skeleton that is deformed to become the mesh structure during expansion. Thus, the deformation rigidity of the connecting line, which is made as a widened part, hardly affects the expansion/contraction deformation of the linear skeleton.

A sixth preferred embodiment provides the stent retriever according to any one of the first through fifth preferred embodiments, wherein the widened section constituting the marker includes a distal end widened part provided at each of multiple locations in the circumferential direction at a distal end of the linear skeleton.

With the stent retriever structured according to the present preferred embodiment, the position of the distal end of the linear skeleton can be ascertained under the X-ray fluoroscopy by viewing the distal end widened parts. In addition, since the distal end widened parts are provided at the multiple locations in the circumferential direction, the diameter of the linear skeleton can be ascertained by viewing the distal end widened parts under the X-ray fluoroscopy to confirm whether the linear skeleton is properly expanding.

A seventh preferred embodiment provides the stent retriever according to any one of the first through sixth preferred embodiments, wherein the linear skeleton of the mesh structure has a plurality of unit cells that are connected to each other in the retriever length direction and in the retriever circumferential direction, and the widened section constituting the marker includes an intercell widened part provided in a portion extending between two adjacent cells of the unit cells in the linear skeleton.

With the stent retriever structured according to the present preferred embodiment, by providing the widened part at the portion extending between the two adjacent unit cells in the linear skeleton, it is not necessary to provide a special portion in the linear skeleton to provide a widened part therein. Thus, the widened section can be arranged with a large degree of freedom and the effect of providing the widened section on the deformation characteristics, etc. of the linear skeleton can be suppressed.

An eighth preferred embodiment provides the stent retriever according to any one of the first through seventh preferred embodiments, wherein the linear skeleton has a circumferential separation portion extending continuously over an entire length in the retriever length direction, and the linear skeleton is unfoldable to both circumferential sides at the circumferential separation portion, and the widened section constituting the marker includes an edge widened part provided at a circumferential edge extending in the length direction along the circumferential separation portion in the linear skeleton.

With the stent retriever structured according to the present preferred embodiment, the circumferential separation portion facilitates expansion/contraction deformation compared to a tubular structure that is continuous over the entire circumference, allowing adaptation to a wide range of vessel sizes, for example.

By providing the edge widened part at the edge along the circumferential separation portion in the linear skeleton, the edge widened part, which is prone to have relatively high deformation rigidity, hardly affects the expansion/contraction and bending deformation characteristics of the linear skeleton. This makes it easier to obtain the linear skeleton with flexible deformation characteristics.

A ninth preferred embodiment provides the stent retriever according to any one of the first through eighth preferred embodiments, wherein the widened section constituting the marker is located on a line spiraling in the retriever length direction.

With the stent retriever structured according to the present preferred embodiment, it is easier to set flexible bending deformation characteristics in the linear skeleton, for example, because the widened section is arranged on a line extending in a spiral shape. Also, the helically extending widened section allows the practitioner to check both the shape of the linear skeleton in the length direction, such as the degree of bending, and the diameter thereof (i.e., the degree of expansion).

A tenth preferred embodiment provides a stent retriever comprising: a self-expansion type linear skeleton that becomes a mesh structure in an expanding state, the linear skeleton of the mesh structure having a plurality of unit cells that are connected to each other in a retriever length direction and in a retriever circumferential direction; a widened section being partially widened in a part constituting the unit cells; and a marker constituted by forming a plating layer on at least one side of the widened section, wherein the widened section constituting the marker is located in a spiral shape as a whole in a continuous state or in a divided and discontinuous state in the retriever length direction.

With the stent retriever structured according to the present preferred embodiment, the widened section constituting the marker is located in the spiral shape as a whole, either in a continuous state or in a divided and discontinuous state in the retriever length direction. This makes it easy to ensure good visibility of the linear skeleton as a whole silhouette under the X-ray fluoroscopy.

An eleventh preferred embodiment provides the stent retriever according to the tenth preferred embodiment, wherein the linear skeleton has a circumferential separation portion extending continuously over an entire length in the retriever length direction, and the linear skeleton is unfoldable to both circumferential sides at the circumferential separation portion.

With the stent retriever structured according to the present preferred embodiment, by adopting a configuration wherein the linear skeleton is unfoldable to the both sides in the circumferential direction at the circumferential separation portion, it is possible to easily perform, for example, the work of forming the plating layer on the inner surface of the widened section in the unfolded state.

A twelfth preferred embodiment of the present invention provides a stent comprising: a self-expansion type linear skeleton that becomes a mesh structure in an expanding state; a widened section being partially widened in the linear skeleton; and a marker constituted by forming a plating layer on both sides of the widened section.

With the stent structured according to this preferred embodiment, as with the stent retriever of the first preferred embodiment described above, it is possible to realize the marker with excellent visibility under the X-ray fluoroscopy while ensuring good mechanical properties such as flexibility and sliding properties of the stent, and also avoiding the risk of the marker falling out.

A thirteenth preferred embodiment of the present invention provides a stent comprising: a self-expansion type linear skeleton that becomes a mesh structure in an expanding state, the linear skeleton of the mesh structure having a plurality of unit cells that are connected to each other in a stent length direction and in a stent circumferential direction; a widened section being partially widened in a part constituting the unit cells; and a marker constituted by forming a plating layer on at least one side of the widened section, wherein the widened section constituting the marker is located in a spiral shape as a whole in a continuous state or in a divided and discontinuous state in the stent length direction.

In the stent structured according to this preferred embodiment, as in the stent retriever according to the tenth preferred embodiment described above, the marker is arranged in the spiral shape utilizing the plurality of unit cells constituting the linear skeleton, which makes it easy to ensure good visibility of the linear skeleton as a whole silhouette under the X-ray fluoroscopy. In the stent of the present preferred embodiment, good mechanical properties are realized owing to the linear skeleton comprising the plurality of unit cells, and the marker can be configured to demonstrate excellent visibility under the X-ray fluoroscopy, while the risk of the marker falling off is avoided.

### EFFECT OF THE INVENTION

According to the present invention, in the stent retriever and the stent, good visibility under the X-ray fluoroscopy owing to the marker can be ensured, while excellent safety can also be achieved by preventing the marker from falling off.

### BRIEF DESCRIPTION OF THE DRAWINGS

FIG. 1 is a perspective view showing a stent retriever as a first practical embodiment of the present invention.
FIG. 2 is a plan view of the stent retriever shown in FIG. 1 in the unfolded and flattened state wherein the stent retriever is unfolded and flattened to both sides in the circumferential direction at a circumferential separation portion.
FIG. 3 is an enlarged cross sectional view taken along line 3-3 of FIG. 2.
FIG. 4 is a perspective view showing a stent retriever as a second practical embodiment of the present invention.
FIG. 5 is a plan view of the stent retriever shown in FIG. 4 in the unfolded and flattened state wherein the stent retriever is unfolded and flattened to both sides in the circumferential direction at a circumferential separation portion.
FIG. 6 is a perspective view showing a stent retriever as a third practical embodiment of the present invention.
FIG. 7 is a plan view of the stent retriever shown in FIG. 6 in the unfolded and flattened state wherein the stent retriever is unfolded and flattened to both sides in the circumferential direction at a circumferential separation portion.
FIG. 8 is a perspective view showing a stent retriever as a fourth practical embodiment of the present invention.
FIG. 9 is a plan view of the stent retriever shown in FIG. 8 in the unfolded and flattened state wherein the stent retriever is unfolded and flattened to both sides in the circumferential direction at a circumferential separation portion.
FIG. 10 is a perspective view showing a stent retriever as a fifth practical embodiment of the present invention.
FIG. 11 is a plan view of the stent retriever shown in FIG. 10 in the unfolded and flattened state wherein the stent retriever is unfolded and flattened to both sides in the circumferential direction at a circumferential separation portion.
FIG. 12 is a perspective view showing a stent retriever as a sixth practical embodiment of the present invention.
FIG. 13 is a plan view of the stent retriever shown in FIG. 12 in the unfolded and flattened state wherein the stent retriever is unfolded and flattened to both sides in the circumferential direction at a circumferential separation portion.
FIG. 14 is a photograph showing stent retrievers used for a visibility confirmation test.
FIG. 15 is a radiograph of the stent retrievers shown in FIG. 14.
FIG. 16 is a plan view showing a stent as a seventh practical embodiment of the present invention in the unfolded and flattened state.

### EMBODIMENTS FOR CARRYING OUT THE INVENTION

There will be described practical embodiments of the present invention, with reference to the drawings.

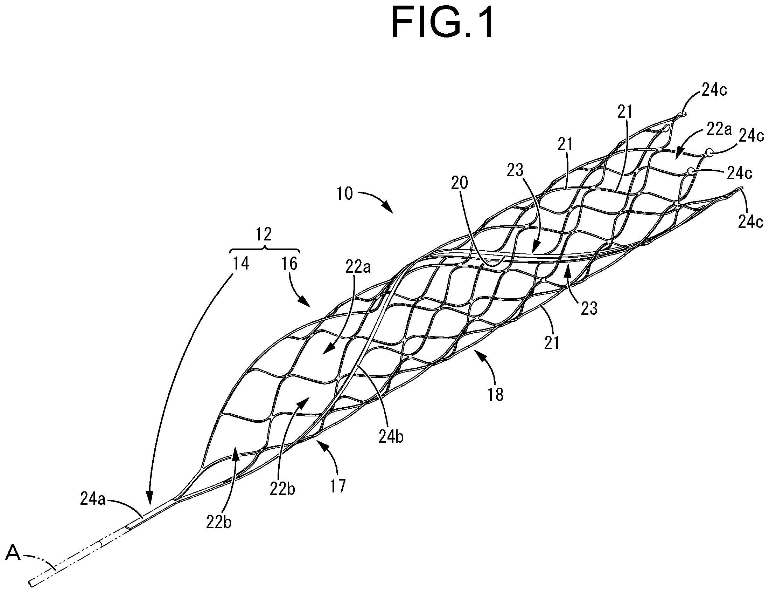

FIG. 1 shows a stent retriever 10 as a first practical embodiment of the present invention. The stent retriever 10 has a linear skeleton 12 that is provided on the distal end side of a control wire A. FIG. 1 shows the stent retriever 10 in an expanding state.

The linear skeleton 12 is formed, for example, of a medical metal, suitably a shape memory alloy such as a Ni-Ti alloy. The linear skeleton 12 is provided integrally with a connecting line 14 connected to the control wire A and a reticular area 16 provided on the distal end side of the connecting line 14 (the distal side during the procedure).

The connecting line 14 has a flat plate shape with the width dimension larger than the thickness dimension, and extends in the retriever length direction. The connecting line 14 is provided as a single line extending straight from the proximal end of the reticular area 16 toward the control wire A, connecting the control wire A to the reticular area 16.

The reticular area 16 is cylindrical as a whole in the expanding state. The proximal end of the reticular area 16 is a curved wall portion 17 with a large partial opening in the circumferential direction in the expanding state, and it is not tubular. The proximal end of the reticular area 16 (the curved wall portion 17) tapers toward the proximal end side in the expanding state and is connected to the single connecting line 14 at its proximal end.

The distal end side of the reticular area 16 relative to the curved wall portion 17 is a tubular circumferential wall portion 18. The tubular circumferential wall portion 18 has a generally cylindrical shape as a whole, and has a circumferential separation portion 20 at a portion in the circumferential direction. The circumferential separation portion 20 extends continuously over the entire length of the tubular circumferential wall portion 18 in the retriever length direction. The tubular circumferential wall portion 18 is divided in the circumferential direction by the circumferential separation portion 20 in a portion in the circumferential direction. As a result, the reticular area 16 including the tubular circumferential wall portion 18 is unfoldable to both sides in the circumferential direction at the circumferential separation portion 20.

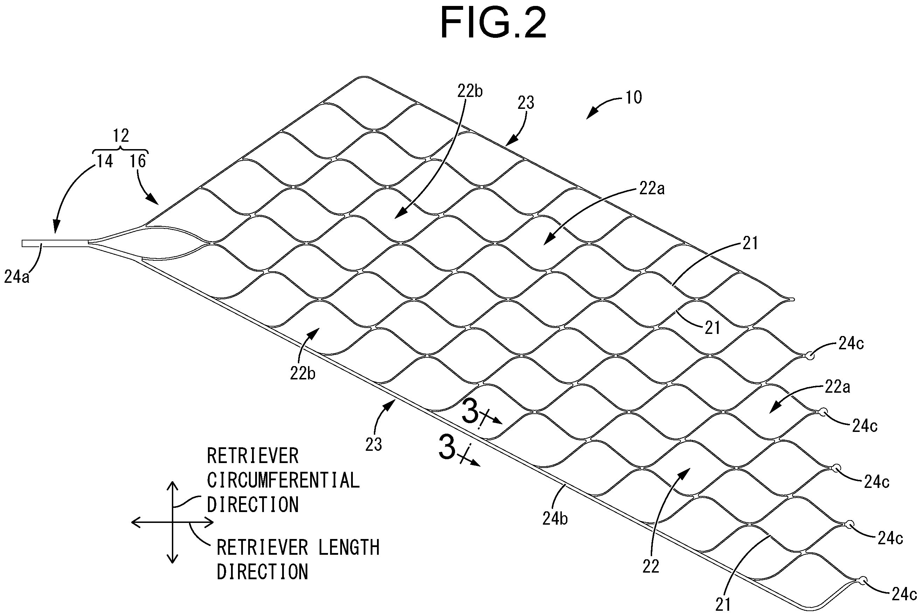

FIG. 2 shows the linear skeleton 12 in an unfolded and flattened state, wherein the reticular area 16 is unfolded to the both sides in the circumferential direction at the circumferential separation portion 20 and laid flat. In FIG. 2, the left-right direction is the retriever length direction, while the up-down direction is the retriever circumferential direction. The retriever length direction is the axial direction of the tubular circumferential wall portion 18 in the expanding state shown in FIG. 1, and the retriever circumferential direction is the circumferential direction of the tubular circumferential wall portion 18 in the expanding state.

As shown in FIG. 2, the reticular area 16 is the unfolded shape of the linear skeleton 12, which spreads from the distal end of the connecting line 14 toward the distal end thereof (to the right side in FIG. 2), in a reticular shape that branches in the retriever circumferential direction, and reaches a predetermined number of stitches, and extends with an approximately constant width. In addition, the linear skeleton 12 has a plurality of wire strips 21 in the reticular area 16 that can be grasped as a waveform continuous in the retriever length direction, with the retriever circumferential direction being the amplitude direction. The wire strips 21, 21 adjacent in the retriever circumferential direction are integrally continuous in the proximity portion of the waveforms. As a result, the linear skeleton 12 in the expanding state is a mesh structure comprising a plurality of unit cells 22 that are connected to each other in the retriever length direction and in the retriever circumferential direction in the reticular area 16.

The plurality of unit cells 22 constituting the mesh structure of the reticular area 16 may be of an approximately single shape and size, or may include different shapes and sizes as needed. In this practical embodiment, the unit cells 22 are formed with an almost constant size as a whole. However, when viewed more precisely, the mesh structure of the reticular area 16 is constituted by including a plurality of unit cells 22a arranged in a row in the retriever length direction and in the retriever circumferential direction, and unit cells 22b, which are arranged between adjacent unit cells 22a and 22a and have a different shape from the unit cells 22a. The reticular area 16 having the mesh structure is a self-expansion type, which can be deformed into the contracted state by the action of an external force, as described below, and recover its shape from the contracted state to the expanding state by the release of the external force, based on superelasticity.

The circumferential separation portion 20 is configured between circumferential edges 23, 23 on the both sides of the linear skeleton 12 (the reticular area 16). The circumferential edges 23 of the linear skeleton 12 extend linearly in a direction inclined to the retriever length direction in the unfolded and flattened state of the linear skeleton 12. The both circumferential edges 23, 23 of the linear skeleton 12 constituting the circumferential separation portion 20 extend generally parallel to each other. Thus, the circumferential separation portion 20 extends in a spiral shape in the expanding state of the linear skeleton 12 shown in FIG. 1.

The linear skeleton 12 has a widened section 24 which is partially widened. The widened section 24 is a part of the linear skeleton 12 that has a larger width dimension than more other parts of the linear skeleton 12. In this practical embodiment, the widened section 24 of the linear skeleton 12 comprises a proximal end widened part 24a set in the connecting line 14, an intermediate widened part 24b serving as an edge widened part provided at one of the circumferential edges 23 of the reticular area 16 that extends out from the connecting line 14 to the distal end of the reticular area 16, and a distal end widened part 24c provided at each distal end of the reticular area 16 and located at each of multiple locations in the circumferential direction.

The proximal end widened part 24a is provided by the connecting line 14 having a flat rectangular plate shape. The proximal end widened part 24a of this practical embodiment extends linearly in the retriever length direction with a roughly constant cross-sectional shape.

The intermediate widened part 24b is wider than the other of the circumferential edges 23 of the reticular area 16 in the linear skeleton 12, and the intermediate widened part 24b is located on a line spiraling along the circumferential separation portion 20 in the retriever length direction, in the expanding linear skeleton 12. The linear skeleton 12 is expanded by superelasticity to a substantially cylindrical shape in the free state in which no external force is exerted. In the expanding state, the intermediate widened part 24b is continuous in the retriever circumferential direction for half the circumference or more of the tubular circumferential wall portion 18, and in this practical embodiment, it extends continuously for an approximately full circumference.

The distal end widened part 24c is located at the distal end of each of the wire strips 21 constituting the plurality of cells in a reticular shape, and is provided at the intersection points closing the distal end side of the cells in the wire strips 21 adjacent to each other in the circumferential direction. Specifically, each of such intersection points is widened by being made into a generally circular disk shape, and each intersection point located at the distal end of the reticular area 16 is made into a wide, approximately curved surface to avoid having a sharp shape, thereby preventing defects such as damaging the blood vessel or catching on the microcatheter described below.

In this practical embodiment, the proximal end widened part 24a, the intermediate widened part 24b, and one of the distal end widened parts 24c are continuous in the retriever length direction, and those widened parts 24a, 24b, 24c constitute a continuous widened part that extends continuously over the entire length in the retriever length direction in the linear skeleton 12.

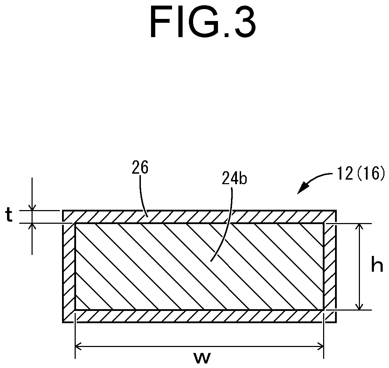

The widened section 24 has a plating layer 26 formed on the both inner and outer circumferential surfaces of the expanding linear skeleton 12, as shown in FIG. 3. The plating layer 26 is formed of a material that is easily distinguishable from the blood vessel and other body tissues by light and dark differences under the X-ray fluoroscopy, such as a radiopaque material. As the forming material of the plating layer 26, a metal material that is not harmful to a human body, such as gold, platinum, palladium, or various alloys of some of them, is used, and the plating layer 26 in this practical embodiment is gold plating. As long as the plating layer 26 is provided on the both sides of the widened section 24, it may be provided only on both sides of the widened section 24 or on the entire surface including the sides of the widened section 24 as shown in FIG. 3. The plating layer 26 is not provided on the wire strips 21, etc. outside the widened section 24, but only on the widened section 24. Since the plating layer 26 is formed on the both sides of the widened section 24, the marker visible under the X-ray fluoroscopy is constituted by the widened section 24 with the plating layer 26.

The thickness dimension t of the plating layer 26 formed on one side of the widened section 24 is suitably 0.003 to 0.015 mm, for example 0.005 mm. The thickness dimension of such plating layer 26 may differ between the inner and outer surfaces of the retriever, but in this practical embodiment, the thickness dimension is the same. The width dimension w of the widened section 24 where the plating layer 26 is to be formed is preferably 0.1 to 0.3 mm, more preferably 0.1 to 0.2 mm, for example 0.2 mm. On the other hand, the width dimension of the wire strips 21 outside the widened section 24 is smaller than the width dimension of the widened section 24, and is suitably 0.05 to 0.1 mm, for example 0.07 mm. The thickness dimension h of the widened section 24 is suitably 0.05 to 0.1 mm, for example, 0.07 mm. In this practical embodiment, the thickness dimension of the linear skeleton 12 that does not include the plating layer 26 is almost constant throughout, but the thickness may be partially different. For example, the proximal end portion including the connecting line 14 may be thicker than other portions.

As the specific manufacturing method of the stent retriever 10, known methods are applicable without limitation. By way of specific examples, the linear skeleton 12 can be manufactured by cutting a metal material made of a Ni-Ti alloy into a predetermined shape by laser or the like. The plating layer 26 can be formed in any portion with any size, for example, by plating while masking. The thickness dimension of the plating layer 26 can be controlled by adjusting the plating conditions, e.g., the plating processing time.

The stent retriever 10 with such a structure is used, for example, to perform thrombus retrieval therapy on a cerebral vascular embolus. First, the stent retriever 10 is inserted into a not-shown microcatheter in a contracted state in which the reticular area 16 is smaller in diameter than in an expanding state. The tubular circumferential wall portion 18 of the stent retriever 10 can be efficiently reduced in diameter by extending in the axial direction since it has the linear skeleton of the mesh structure. In addition to that, the tubular circumferential wall portion 18 in this practical embodiment has a structure that is unfoldable to the both sides in the circumferential direction at the circumferential separation portion 20. This makes it possible that the circumferential portions on the both sides of the circumferential separation portion 20 overlap each other in the thickness direction, and the tubular circumferential wall portion 18 is wound as overlapped, or doubled at least in a portion in the circumferential direction so that the reticular area 16 including the tubular circumferential wall portion 18 can be contracted into a smaller diameter more efficiently.

Since the intermediate widened part 24b on which the plating layer 26 is formed tends to increase the sliding resistance to the microcatheter, it is desirable that when the reticular area 16 gets contracted, one circumferential side of the circumferential separation portion 20 where the intermediate widened part 24b is formed should be involved to be located further on the inner circumferential side than the other circumferential side without the intermediate widened part 24b. This can reduce the sliding resistance to the microcatheter.

The microcatheter containing the stent retriever 10 is then inserted into the vicinity of the thrombus occlusion lesion in the cerebral blood vessel, and the stent retriever 10 protrudes from the microcatheter to the distal side. The stent retriever 10 may protrude from the microcatheter to the distal side by being pushed to the distal side from the microcatheter, or it may protrude from the microcatheter to the distal side by the microcatheter being pulled to the proximal side relative to the stent retriever 10. The reticular area 16 of the stent retriever 10 which protrudes from the microcatheter to the distal side recovers its shape from the contracted state to the expanding state owing to superelasticity. The tubular circumferential wall portion 18 of the reticular area 16, which becomes the mesh structure in the expanding state, bites the thrombus and entangles it.

The stent retriever 10, which has entangled the thrombus, is then housed in a guiding catheter or the like inserted in the carotid artery, for example, by the practitioner pulling the control wire A to the proximal side. This retrieves and removes the thrombus in the blood vessel, thus eliminating the blockage of the cerebral blood vessel caused by the thrombus and quickly restoring the blood flow in the cerebral blood vessel. The thrombus entangled by the stent retriever 10 may be suctioned and removed with a suction catheter if necessary.

According to the stent retriever 10 of this practical embodiment, the linear skeleton 12 is provided with the widened section 24 that is partially widened, and the plating layer 26 is formed on the both sides of the widened section 24, thereby constituting the radiopaque marker. As a result, the stent retriever 10 has good visibility under the X-ray fluoroscopy, and it is possible to easily grasp the relative position of the stent retriever 10 to the microcatheter or the like, and the position and shape, etc. of the stent retriever 10 in the blood vessel.

Since the marker is constituted by the widened section 24 which is partially widened, a large area of radiopaque portion is secured under the X-ray fluoroscopy, thereby improving the visibility of the marker. In addition, by forming the plating layer 26 on the both sides of the widened section 24, the bright/dark ratio (contrast) of the marker is increased under the X-ray fluoroscopy to ensure visibility, that is, the total layer thickness of the plating layer 26 is secured without excessively thickening the plating layer 26 on each side of the widened section 24. Also, it is easy to maintain a good balance between the wire strips 21 and the plating layer 26, etc. by the difference in materials. Moreover, thickening the plating layer 26 requires the width dimension of the widened section 24 that serves as the base material. However, by providing the plating layer 26 on the both sides to avoid thickening of each plating layer 26, it is also possible to set the thickness dimension of the entire plating layer 26 to be large while avoiding adverse effects on the deformation characteristics of the stent retriever 10 caused by excessive widening of the widened section 24.

The stent retriever 10 of this practical embodiment has the proximal end widened part 24a provided at the proximal end of the linear skeleton 12, the intermediate widened part 24b continuous over the entire length of the reticular area 16 in the length direction, and the distal end widened part 24c provided at the distal end of the linear skeleton 12, so that under the X-ray fluoroscopy the entire stent retriever 10 is easy to grasp. That is, for example, when the stent retriever 10 is pushed to the distal side out of the microcatheter, the amount of protrusion of the stent retriever 10 from the distal end of the microcatheter can be grasped owing to the proximal end widened part 24a. Also, owing to the intermediate widened part 24b, it is possible to grasp the shape in which the reticular area 16 is arranged, for example. Moreover, for example, when the stent retriever 10 is being retracted into the guiding catheter, it is possible to ascertain the extent to which it is retracted, owing to the distal end widened parts 24c.

Since the intermediate widened part 24b extends in a spiral shape, the shape of the reticular area 16, including the degree of expansion and the like, can be grasped more easily and accurately. Furthermore, since the proximal end widened part 24a, the intermediate widened part 24b, and the distal end widened part 24c are provided continuously over the entire length of the linear skeleton 12, the linear skeleton 12 can be easily grasped under the X-ray fluoroscopy.

The intermediate widened part 24b is provided at the circumferential edge 23 of the linear skeleton 12 extending in the length direction along the circumferential separation portion 20. This suppresses the restraint of the wire strips 21 by the intermediate widened part 24b, whose width tends to increase the deformation rigidity, so that the deformation rigidity of the linear skeleton 12 can be suppressed and flexible deformation characteristics are easily obtained.

Since the distal end widened parts 24c are provided at the multiple locations in the circumferential direction, the degree of expansion of the tubular circumferential wall portion 18 and the like can be determined by checking the arrangement of the plurality of distal end widened parts 24c under the X-ray fluoroscopy.

The marker, which is visible under the X-ray fluoroscopy, is constituted by forming the plating layer 26 on the widened section 24 provided on the linear skeleton 12, so that the marker is provided integrally with the linear skeleton 12. Therefore, even if the marker is caught on the inner circumferential surface of the microcatheter or on the vessel wall, for example, the marker more seldom falls off from the linear skeleton 12 than when a separate marker is attached to the linear skeleton, and a higher level of safety can be achieved. In addition, the marker does not need to be prepared as a separate component from the linear skeleton 12, and thus the number of parts can be reduced.

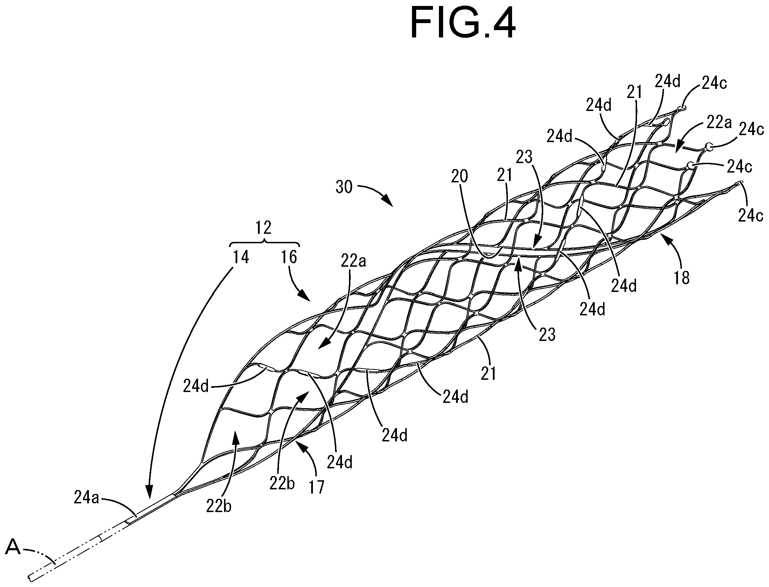

FIG. 4 shows a stent retriever 30 as a second practical embodiment of the present invention. In the following description, explanation about components and parts that are substantially the same as those of the first practical embodiment may be omitted, by providing the same symbols in the drawings.

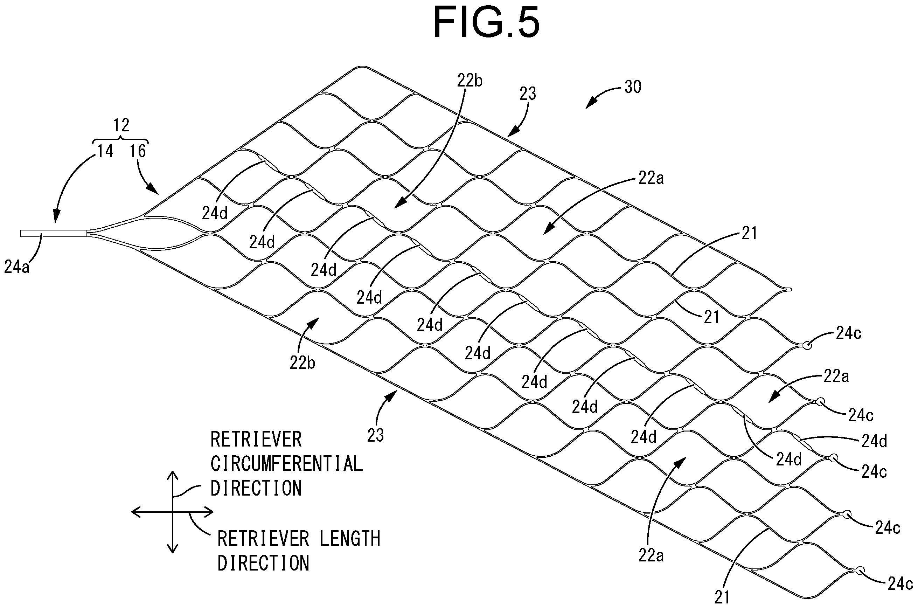

The stent retriever 30 does not have the intermediate widened part 24b along the circumferential separation portion 20, as also shown in the net of FIG. 5. The stent retriever 30 has a plurality of intermediate widened parts 24d in the middle portion of the reticular area 16 in the retriever length direction. The intermediate widened parts 24d are intercell widened parts provided in the wire strips 21 extending between adjacent unit cells 22, 22. The intermediate widened part 24d is in an elongated shape shorter than the portion extending between the unit cells 22, 22 in the wire strip 21, and is arranged on a spiral line in the expanding reticular area 16. The intermediate widened part 24d is a divided widened part provided separately at multiple locations in the retriever length direction without being continuous over the entire length in the retriever length direction.

According to the stent retriever 30 with the structure according to this practical embodiment, the plurality of intermediate widened parts 24d can ensure good visibility of the reticular area 16 under the X-ray fluoroscopy. Since the plurality of intermediate widened parts 24d are arranged in a spiral shape inclined to the retriever length direction, the shape of the expanding reticular area 16 is easy to grasp.

In this practical embodiment, since the plurality of intermediate widened parts 24d are intermittently arranged in the retriever length direction, the effect of the intermediate widened parts 24d on the deformation rigidity of the reticular area 16 can be suppressed compared to when they are continuous over the entire length in the retriever length direction. This makes it possible to obtain the stent retriever 30 with excellent followability in relation to the curved shape of the blood vessel, etc. In particular, the absence of the band-shaped intermediate widened part 24b at the circumferential edge 23 of the tubular circumferential wall portion 18 along the circumferential separation portion 20 easily allows relative displacement of the both circumferential portions of the tubular circumferential wall portion 18. This makes it possible to set more flexible curvature deformation characteristics for the tubular circumferential wall portion 18.

In this practical embodiment, as an example of the arrangement of the intermediate widened parts 24d, there is shown the arrangement thereof on a line extending in a spiral shape. However, the intermediate widened part 24d can be provided on the portion of any wire strip 21 extending between the unit cells 22, 22, and the intermediate widened part 24d can be arranged with a large degree of freedom.

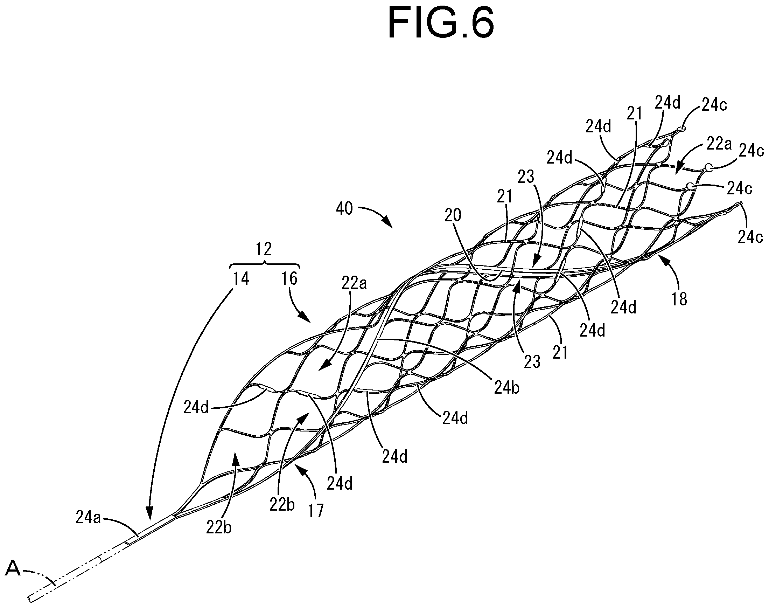

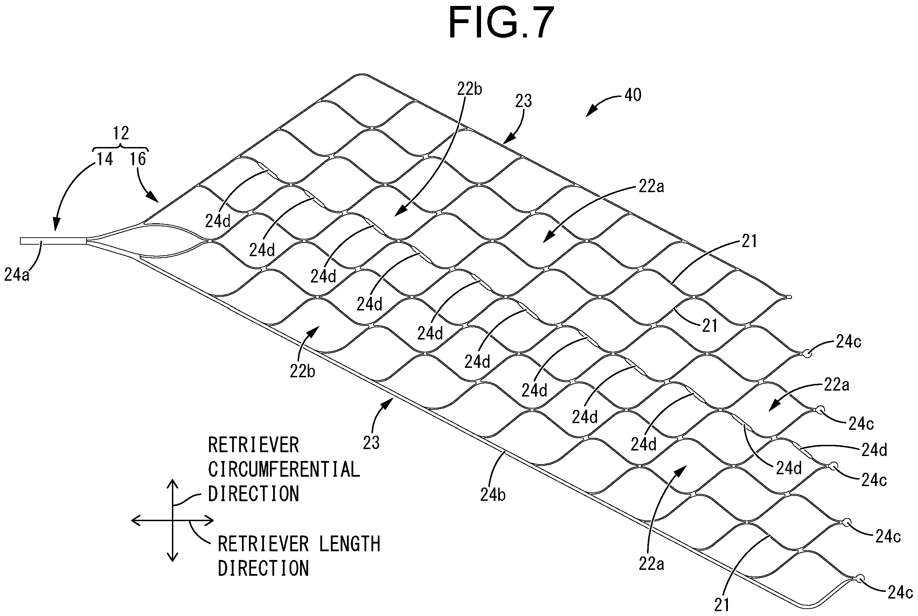

Like a stent retriever 40 as a third practical embodiment shown in FIGS. 6 and 7, the stent retriever may include both the intermediate widened part 24b shown in the first practical embodiment and the intermediate widened part 24d shown in the second practical embodiment. According to this, better visibility under the X-ray fluoroscopy can be achieved while obtaining the flexible deformation characteristics required for the tubular circumferential wall portion 18.

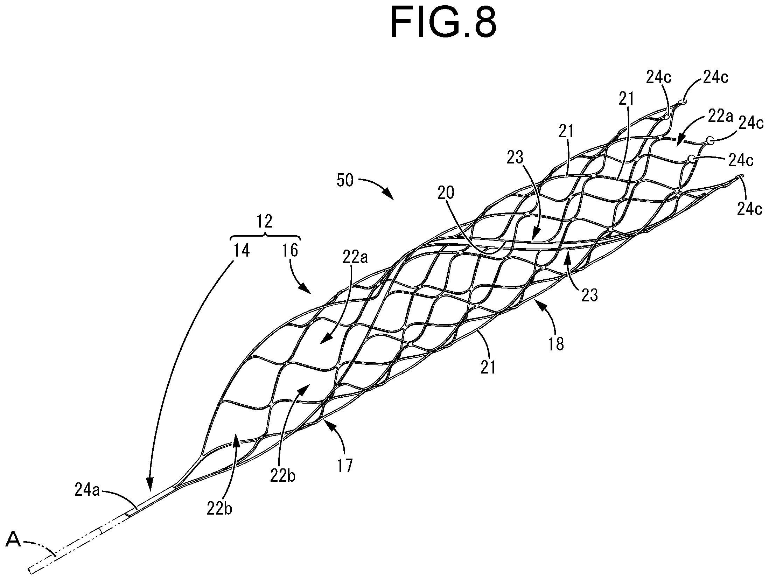

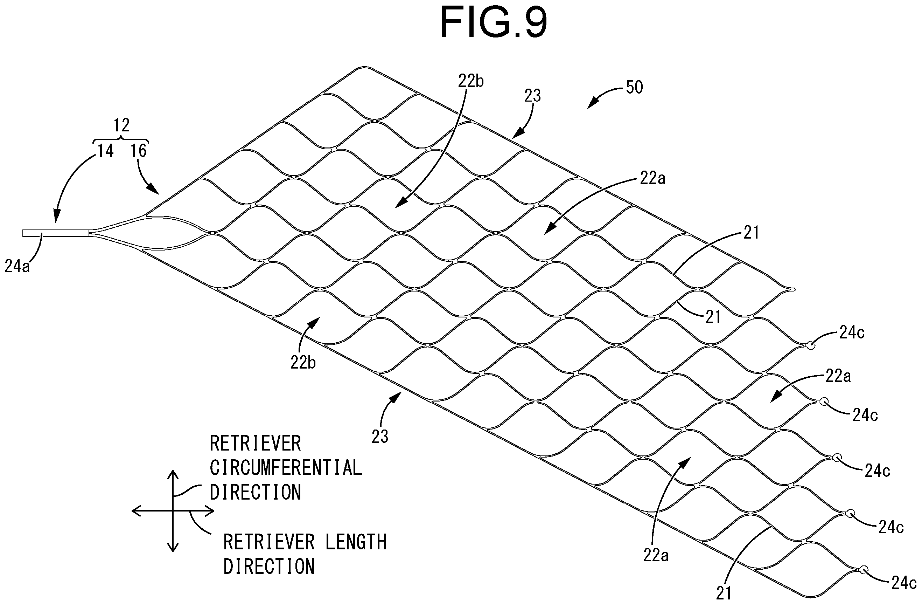

FIGS. 8 and 9 show a stent retriever 50 as a fourth practical embodiment. The stent retriever 50 has, as the widened section 24, the proximal end widened part 24a by the connecting line 14 and the distal end widened part 24c provided at the distal end of the reticular area 16. The middle portion of the reticular area 16 is not provided with any widened part.

According to the stent retriever 50 of this practical embodiment, the absence of the widened section 24 in any portion of the linear skeleton 12 other than the proximal end and the distal end realizes the flexible deformation characteristics of the tubular circumferential wall portion 18 more effectively. Additionally, the visibility of the stent retriever 50 is ensured by the widened parts 24a and 24c, at the proximal end and the distal end of the stent retriever 50 for which the visibility under the X-ray fluoroscopy is required especially.

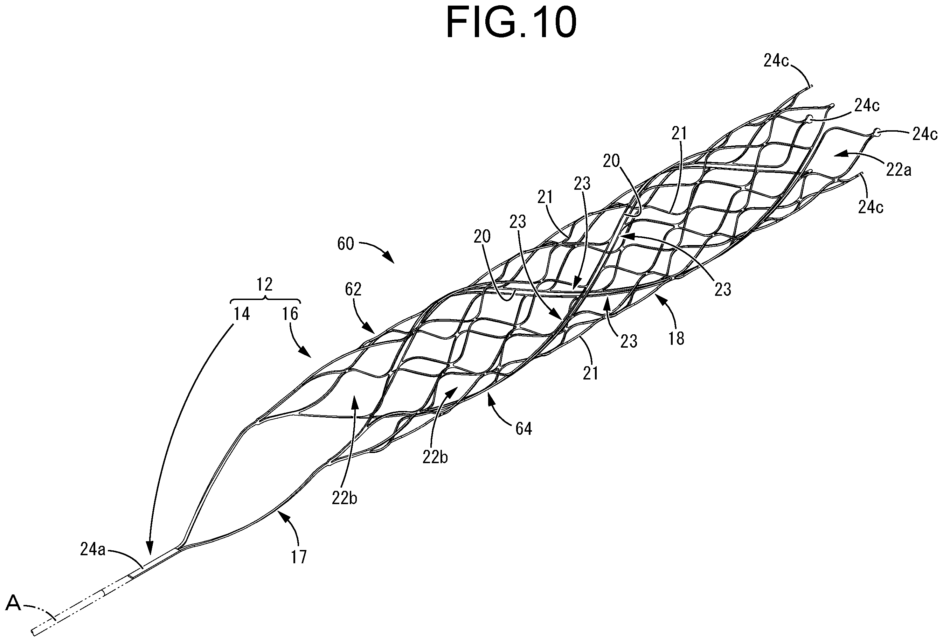

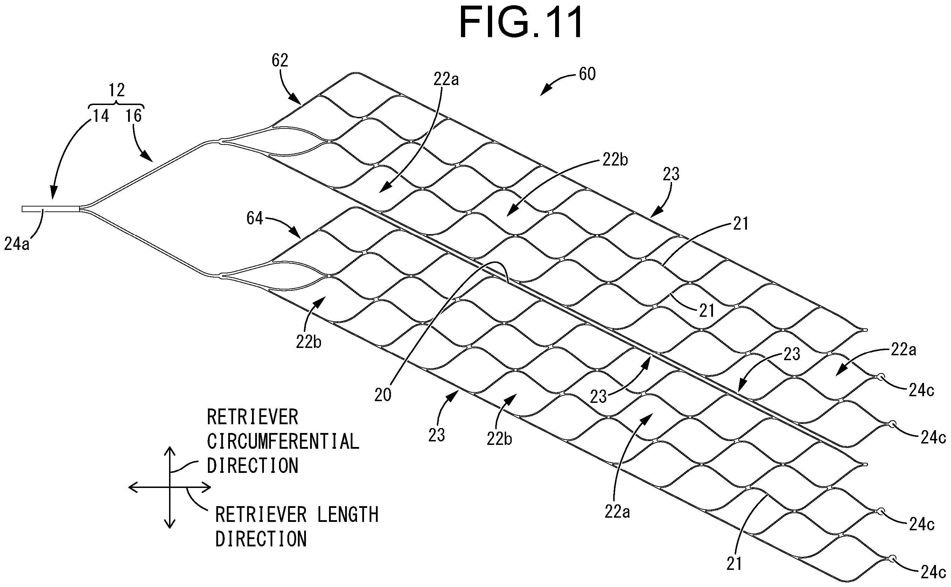

FIG. 10 shows a stent retriever 60 as a fifth practical embodiment. The stent retriever 60 has two circumferential separation portions 20, 20 extending in a double-helix shape. Specifically, as shown in the net of FIG. 11, the reticular area 16 is composed of a first cell group 62 and a second cell group 64, each comprising a plurality of unit cells 22.

The first cell group 62 and the second cell group 64 have each circumferential edge 23 extending linearly at an angle to the retriever length direction, and each circumferential edge 23 is spaced apart from each other and approximately parallel to each other. A first circumferential edge 23 of the first cell group 62 and a second circumferential edge 23 of the second cell group 64 are mutually separated but located close, and one circumferential separation portion 20 is formed between the first circumferential edge 23 of the first cell group 62 and the second circumferential edge 23 of the second cell group 64. A second circumferential edge 23 of the first cell group 62 and a first circumferential edge 23 of the second cell group 64 are mutually separated but located close in the expanding state in which the reticular area 16 is made tubular, and the other circumferential separation portion 20 is formed between the second circumferential edge 23 of the first cell group 62 and the first circumferential edge 23 of the second cell group 64. The other circumferential separation portion 20 is formed between the second circumferential edge 23 of the first cell group 62 and the first circumferential edge 23 of the second cell group 64 (see FIG. 10).

In this practical embodiment, only the distal end widened part 24c is provided as the marker in the first and second cell groups 62, 64 constituting the reticular area 16. Alternatively, it is also possible to provide the intermediate widened part 24b extending along the circumferential separation portion 20 and/or the intermediate widened part 24d as the intercell widened part arranged between the adjacent unit cells 22, 22. In the case of providing the intermediate widened part 24b, for example, two intermediate widened parts 24b, 24b can be provided along the two circumferential separation portions 20, 20, which improves the visibility of the reticular area 16 under the X-ray fluoroscopy owing to the double-helix marker.

According to the stent retriever 60 with such a structure, the two circumferential separation portions 20, 20 make it easier to set the deformation characteristics of the tubular circumferential wall portion 18 more flexible. In particular, since the two circumferential separation portions 20, 20 are provided in a double-helix shape, the curvature and expansion/contraction deformation characteristics of the tubular circumferential wall portion 18 can be made more flexible, thereby improving followability to the curved portion of the blood vessel and accommodating to a wide range of vessel sizes owing to expandability in the occluded portion, and the like.

The two circumferential separation portions 20, 20 need not have the same circumferential amplitude as each other, but may be formed with different amplitudes. Alternatively, it is also possible to three or more circumferential separation portions 20.

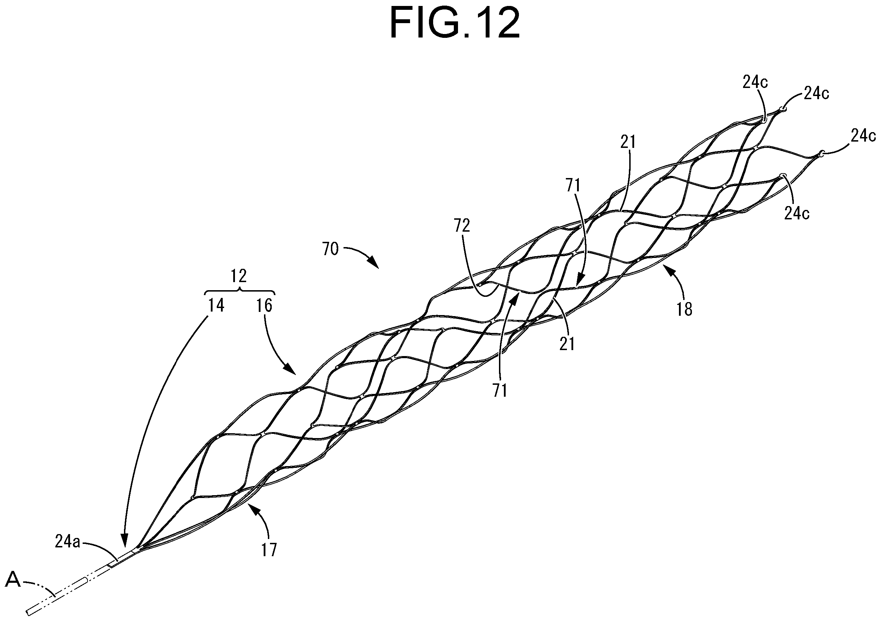

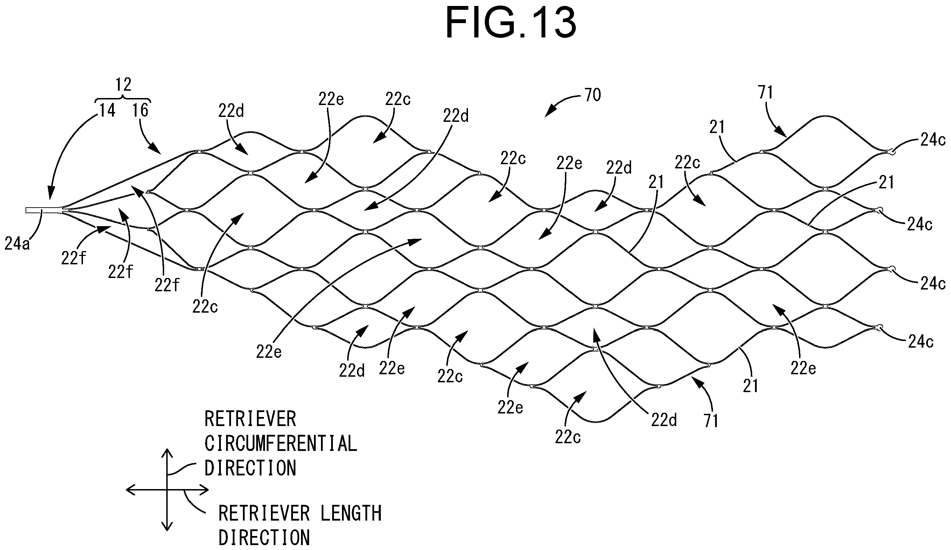

FIG. 12 shows a stent retriever 70 as a sixth practical embodiment. In the stent retriever 70, any circumferential edges 71, 71 on both sides of the reticular area 16 do not have a straight line shape, as shown in the net of FIG. 13. As a result, a circumferential separation portion 72 shown in FIG. 12, which is composed of the circumferential edges 71, 71 of the reticular area 16, has a width dimension in the retriever circumferential direction varying in the retriever length direction, while having a wavy shape that goes back and forth in the circumferential direction, not a spiral shape, as a whole.

The circumferential separation portion 72 extends in the retriever length direction while inclining in the retriever circumferential direction. The circumferential separation portion 72 has a retriever circumferential amplitude larger than the circumferential size of the unit cell 22 in the tubular circumferential wall portion 18. In this practical embodiment, the circumferential amplitude of the circumferential separation portion 72 is less than half the circumference of the tubular circumferential wall portion 18.

As shown in FIG. 13, the reticular area 16 has a mesh structure in which unit cells 22c and 22d, whose width dimensions in the retriever circumferential direction (circumferential size) differ from each other, are arranged alternately in the retriever length direction, and a unit cell 22e is provided adjacent to the sides of the adjacent unit cells 22c and 22d in the retriever length direction. The proximal end of the reticular area 16 that connects with the connecting line 14 (the curved wall portion 17) is composed of three thin unit cells 22f that are elongated in the retriever length direction. The circumferential amplitude of the circumferential separation portion 72 is larger than any of the circumferential sizes of the unit cells 22c, 22d, 22e, 22f.

As can be understood in the light of the stent retriever 70 with this construction, the specific shape of the circumferential separation portion provided in the tubular circumferential wall portion 18 is not particularly limited. It is not necessary that the circumferential separation portion extends in a spiral shape, nor that the width dimension in the retriever circumferential direction is approximately constant in the retriever length direction. The specific shape and size of the unit cells constituting the mesh structure of the tubular circumferential wall portion 18 are set based on the required deformation characteristics, containing characteristics, and friction characteristics of the tubular circumferential wall portion 18, and the like and they are not particularly limited.

In the stent retriever 70 shown in FIGS. 12 and 13, the marker can be constituted by providing the intermediate widened part 24b extending along the circumferential separation portion 72, or the intermediate widened part 24d between adjacent unit cells 22, 22.

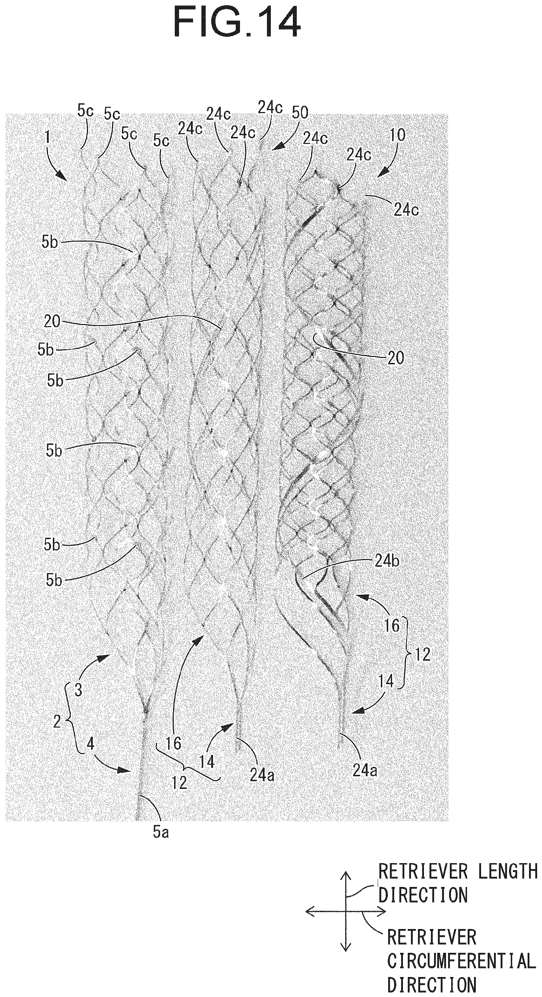

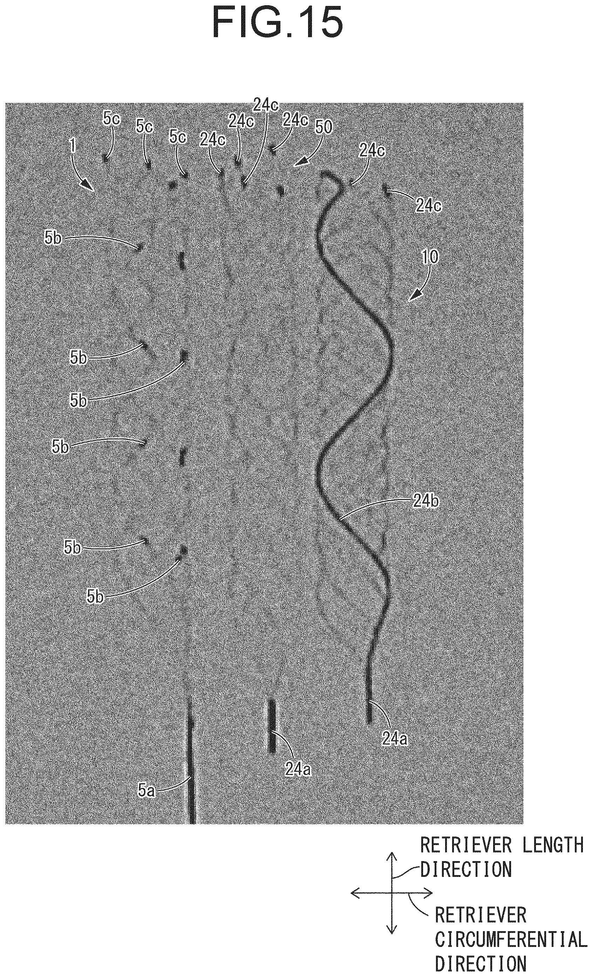

By the way, as shown in FIGS. 14 and 15, the inventor performed a visibility confirmation test to check the visibility of the stent retrievers under the X-ray fluoroscopy. FIG. 14 is a photograph of the stent retrievers used in the visibility confirmation test, and the three stent retrievers 10, 50, 1 are shown in a row from right to left in the drawing. In the following explanation of the stent retrievers used in the experiments, the same symbols may be applied to the parts and components that correspond to those of the practical embodiments described above, and the explanation of the parts and components may be omitted.

The stent retriever 10 on the right side of the drawing has a structure relating to the present invention and corresponding to the first practical embodiment described above. Specifically, the stent retriever 10 is provided with the proximal end widened part 24a constituted by the connecting line 14, the spiral-shaped intermediate widened part 24b extending along the circumferential separation portion 20, and the distal end widened part 24c provided at the distal end thereof. The radiopaque marker is formed by forming the gold plating layer 26 on the both sides of each of the widened parts 24a, 24b, and 24c.

The stent retriever 50 at the center of the drawing in the left-right direction has a structure relating to the present invention and corresponding to the fourth practical embodiment described above. Specifically, the stent retriever 50 is provided with the proximal end widened part 24a constituted by the connecting line 14 and the distal end widened part 24c provided at the distal end thereof. The radiopaque marker is formed by forming the gold plating layer 26 on the both sides of each of the widened parts 24a, 24c.

A stent retriever 1 on the left side of the drawing is a conventional product and is equipped with a linear skeleton 2. The linear skeleton 2 has a reticular area 3, which is a mesh structure in an expanding state. To the reticular area 3, a proximal end connecting line 4 that extends toward the proximal end and is connected to the control wire A is attached. The connecting line 4 is a radiopaque proximal end marker 5a, which is attached to the proximal end of the reticular area 3 by means of swaging or the like. A plurality of intermediate markers 5b, arranged at predetermined intervals in the retriever length direction, are attached to the reticular area 3 by means of swaging or the like. At the distal end of the reticular area 3, some distal end markers 5c are attached at multiple locations in the circumferential direction, by means of swaging or the like. The markers 5a, 5b, and 5c are all made of platinum-iridium alloy (Pt-Ir) and are separate from the Ni-Ti alloy linear skeleton 2.

FIG. 15 shows radiographs of the three stent retrievers 10, 50, 1. FIG. 15 confirms that for any of the stent retrievers 10, 50, 1, the visibility of the markers is ensured and that the distal end and proximal end of the stent retrievers 10, 50, 1 are visible under the X-ray fluoroscopy.

In addition, the stent retriever 10 on the right side of the drawing has a band-shaped marker constituted by the intermediate widened part 24b of the reticular area 16. This makes it easier to grasp the position and shape of the reticular area 16 compared to the conventional stent retriever 1, which has a spot-shaped marker.

In the conventionally structured stent retriever 1 on the left side of the drawing, a separate marker 5 is attached to the linear skeleton 2 by means of swaging, soldering, or the like, and there is a risk that the marker 5 may fall off the linear skeleton 2. On the other hand, the stent retriever 10 on the right side of the drawing and the stent retriever 50 at the center of the drawing in the left-right direction have the marker constituted by the widened section 24 with the plating layer 26 formed thereon, and since the marker is integrally provided on the linear skeleton 12, there is no risk that the marker falls off the linear skeleton 12.

As described above, experiments have confirmed that the stent retrievers 10, 50 of the present invention, which have no risk of marker dropout, have comparable or even better visibility under the X-ray fluoroscopy than the stent retriever 1 of conventional construction.

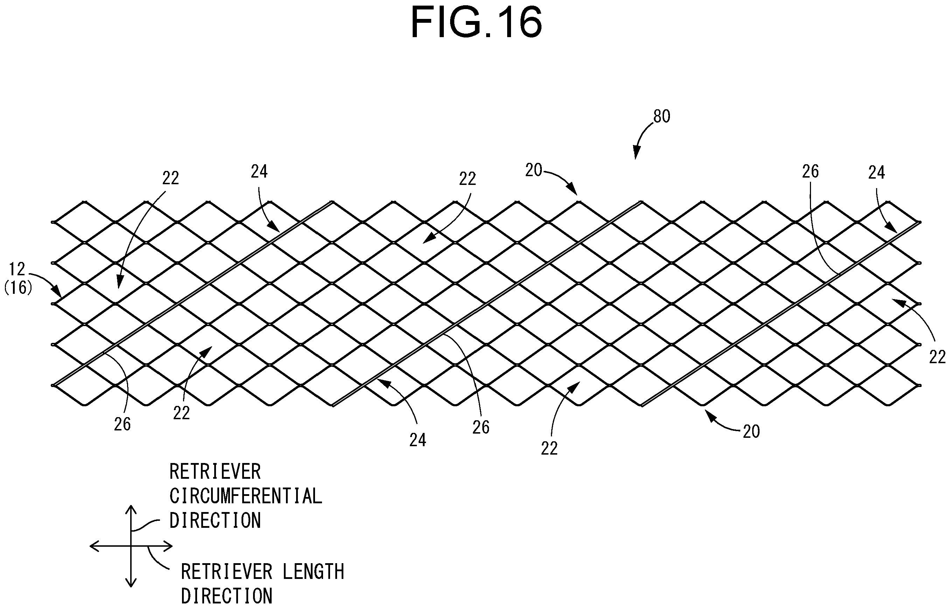

FIG. 16 shows a stent 80 as a seventh practical embodiment of the invention. FIG. 16 shows the stent 80, as a net corresponding to FIG. 2, and like the product shape diagram of FIG. 1 relative to the net of FIG. 2, the stent is shape-memorized so that in the expanding state of the product, it is rounded in the up-down direction in the drawing to become a tubular stent as a whole. In this practical embodiment, for ease of understanding, to the components and parts corresponding to those of the first practical embodiment shown in FIGS. 1 to 3, the same symbols as in the first practical embodiment are applied in the drawing.

The stent 80 of this practical embodiment has the linear skeleton 12 made of a shape memory alloy, and is shape-memorized to a cylindrical shape as a whole in the stent expanding state. Specifically, the linear skeleton 12 has the circumferential separation portion 20 extending continuously over the entire length in the length direction, in a circumferential portion of the stent 80. The linear skeleton 12 can be unfolded to both sides in the circumferential direction at such circumferential separation portion 20 to be in the unfolded and flattened state shown in FIG. 16. The circumferential separation portions 20, 20 on the both sides in the circumferential direction are butted against each other owing to the shape memory action to become cylindrical as a whole in a stent expanding state.

The linear skeleton 12 in the expanding state, like the stent retriever of the first practical embodiment, comprises a self-expansion type reticular area 16 having a mesh structure. This reticular area 16 has a plurality of unit cells 22 connected to each other in the circumferential direction and the length direction of the stent 80.

The linear skeleton 12 has the widened section 24 that is partially widened. In this practical embodiment, the widened section 24 is provided extending so as to connect specific sides of consecutive unit cells, so that the widened section 24 is arranged in a spiral line in the stent length direction in the expanding linear skeleton 12. In the unfolded and flattened state of FIG. 16, the widened section 24 is interrupted at the top and bottom edges, but in the expanding cylindrical state, the top and bottom edges of the widened parts 24, 24 each extending diagonally, which are located at the circumferential separation portion 20, are connected by mutually approaching or touching each other. As a result, the widened section 24 is formed to extend in a spiral, continuous manner over the entirety in the length direction of the stent, as a whole.

In the widened section 24, the plating layer 26 is formed on either or both of the inner circumferential surface and the outer circumferential surface of the linear skeleton 12 in the expanding state. The material and thickness dimensions, etc. of such plating layer should be in accordance with the mode of the first practical embodiment described above.

The stent 80 of this practical embodiment is used for treatment, for example, by being delivered in a contracted state to a lesion site or the like in a blood vessel using a delivery catheter, and indwelling temporarily or for a long period of time in a shape-recovered state. In such a case, the stent 80 of this practical embodiment can ensure good mechanical properties such as flexibility and obtain good visibility under the X-ray fluoroscopy owing to the widened section 24 with the plating layer 26, and enjoy the same effect as the stent retriever of the first practical embodiment.

Although the above practical embodiments of the present invention have been described in detail, the invention is not limited by that specific description. For example, it is desirable that the widened part 24 provided in the middle of the reticular area 16 has an elongated shape extending over a predetermined length, like the intermediate widened part 24b and the intermediate widened part 24d shown in the aforesaid practical embodiments, as it is easier to grasp the shape of the reticular area 16, but it may have a spot-like shape.

For example, when the marker is formed by the intermediate widened part 24b extending over almost the entire length of the reticular area 16, it is also possible to omit at least one of the marker by the proximal end widened part 24a and the marker by the distal end widened part 24c. In addition, in configuring the marker extending over the entire length in the retriever length direction according to the second preferred embodiment, the marker will do as long as the stent retriever can be checked over substantially its entire length in the length direction. This includes modes in which small portions such as the ends or the center do not constitute markers, partially, due to manufacturing conditions for example.

In configuring the marker in a divided mode according to the third preferred embodiment, the intermediate widened parts 24d as divided widened parts provided separately at the multiple locations in the retriever length direction is shown as an example in the aforesaid practical embodiment. However, such divided widened parts may be provided separately at multiple locations, or independently of each other in the circumferential direction.

In the above-mentioned practical embodiment, the plurality of wire strips 21, which can be grasped with a waveform extending in the retriever length direction while curving in reciprocation in the retriever circumferential direction, are arranged in the wave amplitude direction. The phases of the adjacent wire strips 21, 21 are shifted mutually in the wave length direction, and the approximate tops of the waves adjacent to each other are joined as the joining point, thereby forming the unit cells 22. However, the shape of the unit cell 22 is appropriately designed according to the required deformation characteristics and thrombus trapping performance, and the like and it is not particularly limited. Alternatively, the unit cell can be formed by arranging linear zigzag-shaped wire strips instead of the wave-form wire strips. The deformation characteristics of the reticular area 16 can also be adjusted by arranging larger unit cells, for example, at the proximal end of the reticular area 16 (the curved wall portion 17), where the effect on the thrombus trapping performance is small. Thus, the size of the unit cells constituting the mesh structure need not be approximately constant in shape and size throughout, but cells of different shapes and sizes can be employed in parts, and the width dimension of the linear skeleton can be different for only the part constituting a particular cell, compared to other parts.

Although the unit cell 22 of the aforementioned practical embodiment is made as a closed cell by making the approximate tops of the waves of the adjacent wire strips 21, 21 as the joining points, the unit cell may be an open cell. Specifically, the unit cell is an open cell, for example when the linear skeleton has a plurality of annular bodies that extend in the circumferential direction while waving or zigzagging in the axial direction, and has a structure wherein the annular bodies separated in the axial direction are partially connected in the circumferential direction.

The circumferential separation portion 20 is not essential in the reticular area 16, and the reticular area 16 may have a tubular shape continuous around its entire circumference. The circumferential separation portion 20 may, for example, extend in a straight or wavy mode, being approximately parallel to the retriever length direction instead of inclining at a fixed angle.

In the practical embodiment described above, a structure in which the plating layer 26 is provided on the both sides of the wire strip 21 is shown as an example. However, the plating layer 26 may be provided on only one side of the wire strip 21. When the plating layer 26 is provided on only one side of the wire strip 21, it is suitable to provide the plating layer 26 on the inner surface of the wire strip 21 so that the slipperiness of the outer surface of the wire strip 21 against the living body can be avoided from deteriorating due to the plating layer 26, etc.

In particular, when the plating layer 26 is formed on only one side of the wire strip 21, the widened section 24 constituting the marker is located in a spiral shape as a whole, in a continuous state (FIG. 2) or in a divided and discontinuous state (FIG. 5) in the retriever length direction, thus making it easy to confirm the entire image of the wire strip 21 with good visibility under the X-ray fluoroscopy.

When forming the plating layer 26 on only one side of the wire strip 21, the plating layer 26 can be easily formed on the inner surface of the wire strip 21 in the unfolded state of the linear skeleton 12, by adopting a configuration in which the linear skeleton 12 is unfoldable to the both sides in the circumferential direction at the circumferential separation portion 20.

In addition, extension modes related to the aforesaid practical embodiments and the like are not limited to the stent retriever, but they may be applied to the stent illustrated in FIG. 16, as well. Also, the specific shape and structure of the linear skeleton and the reticular area, the position and configuration of the widened section, and the like in the stent are not limited to the practical embodiment described above, but they can be implemented in various modes, like the stent retriever.

### KEYS TO SYMBOLS

10: Stent retriever (first practical embodiment); 12: Linear skeleton; 14: Connecting line; 16: Reticular area; 17: Curved wall portion; 18: Tubular circumferential wall portion; 20: Circumferential separation portion; 21: Wire strip; 22: Unit cell; 22a: Unit cell; 22b: Unit cell; 22c: Unit cell; 22d: Unit cell; 22e: Unit cell; 22f: Unit cell; 23: Circumferential edge; 24: Widened section; 24a: Proximal end widened part; 24b: Intermediate widened part (edge widened part); 24c: Distal end widened part; 24d: Intermediate widened part (intercell widened part, divided widened part); 26: Plating layer; 30: Stent retriever (second practical embodiment); 40: Stent retriever (third practical embodiment); 50: Stent retriever (fourth practical embodiment); 60: Stent retriever (fifth practical embodiment); 62: First cell group; 64: Second cell group; 70: Stent retriever (sixth practical embodiment); 71: Circumferential edge; 72: Circumferential separation portion; 80: Stent; 1: Stent retriever (conventional product); 2: Linear skeleton; 3: Reticular area; 4: Connecting line; 5: Marker; 5a: Proximal end marker; 5b: Intermediate marker; 5c: Distal end marker; A: Control wire

## Claims

1. A stent retriever comprising:
a self-expansion type linear skeleton that becomes a mesh structure in an expanding state;
a widened section being partially widened in the linear skeleton; and
a marker constituted by forming a plating layer on both sides of the widened section.

2. The stent retriever according to claim 1, wherein the widened section constituting the marker includes a continuous widened part extending continuously over an entire length of the liner skeleton in a retriever length direction.

3. The stent retriever according to claim 1 or 2, wherein the widened section constituting the marker includes a plurality of divided widened parts provided separately at multiple locations.

4. The stent retriever according to any one of claims 1-3, wherein the widened section constituting the marker extends continuously in a retriever circumferential direction for half a circumference or more.

5. The stent retriever according to any one of claims 1-4, wherein
at a proximal end of the linear skeleton, a single connecting line extends toward a control wire, and
the widened section constituting the marker includes a proximal end widened part constituted by the connecting line.

6. The stent retriever according to any one of claims 1-5, wherein the widened section constituting the marker includes a distal end widened part provided at each of multiple locations in the circumferential direction at a distal end of the linear skeleton.

7. The stent retriever according to any one of claims 1-6, wherein
the linear skeleton of the mesh structure has a plurality of unit cells that are connected to each other in the retriever length direction and in the retriever circumferential direction, and
the widened section constituting the marker includes an intercell widened part provided in a portion extending between two adjacent cells of the unit cells in the linear skeleton.

8. The stent retriever according to any one of claims 1-7, wherein
the linear skeleton has a circumferential separation portion extending continuously over an entire length in the retriever length direction, and the linear skeleton is unfoldable to both circumferential sides at the circumferential separation portion, and
the widened section constituting the marker includes an edge widened part provided at a circumferential edge extending in the length direction along the circumferential separation portion in the linear skeleton.

9. The stent retriever according to any one of claims 1-8, wherein the widened section constituting the marker is located on a line spiraling in the retriever length direction.

10. A stent retriever comprising:
a self-expansion type linear skeleton that becomes a mesh structure in an expanding state, the linear skeleton of the mesh structure having a plurality of unit cells that are connected to each other in a retriever length direction and in a retriever circumferential direction;
a widened section being partially widened in a part constituting the unit cells; and
a marker constituted by forming a plating layer on at least one side of the widened section, wherein
the widened section constituting the marker is located in a spiral shape as a whole in a continuous state or in a divided and discontinuous state in the retriever length direction.

11. The stent retriever according to claim 10, wherein the linear skeleton has a circumferential separation portion extending continuously over an entire length in the retriever length direction, and the linear skeleton is unfoldable to both circumferential sides at the circumferential separation portion.

12. A stent comprising:
a self-expansion type linear skeleton that becomes a mesh structure in an expanding state;
a widened section being partially widened in the linear skeleton; and
a marker constituted by forming a plating layer on both sides of the widened section.

13. A stent comprising:
a self-expansion type linear skeleton that becomes a mesh structure in an expanding state, the linear skeleton of the mesh structure having a plurality of unit cells that are connected to each other in a stent length direction and in a stent circumferential direction;
a widened section being partially widened in a part constituting the unit cells; and
a marker constituted by forming a plating layer on at least one side of the widened section, wherein
the widened section constituting the marker is located in a spiral shape as a whole in a continuous state or in a divided and discontinuous state in the stent length direction.
